# EUROPÄISCHE PATENTSCHRIFT

(11) **EP 0 336 000 B1**
(45) Veröffentlichungstag und Bekanntmachung des Hinweises auf die Patenterteilung: **17.03.1993**
(21) Anmeldenummer: 88105632.9
(22) Anmeldetag: 08.04.1988
(51) Int. Cl.: A61K 9/10, A61K 47/00, A61K 7/00

(54) **Ambiphile Creme**
Ambiphilic cream
Crème ambiphile

(43) Veröffentlichungstag der Anmeldung: 11.10.1989
(73) Patentinhaber: STADA ARZNEIMITTEL AG, D-61116 Bad Vilbel (DE)
(72) Erfinder: Nürnberg, Eberhard, Prof. Dr., D-8525 Uttenreuth/Weiher (DE); Leonhard, Uwe, D-8671 Toepen (DE); Hornstein, Otto Paul, Prof. Dr. med., D-8525 Uttenreuth (DE); Griessmayer, Gabriele, Dr. med., D-8520 Erlangen (DE); Perschbacher, Harald, Dr., D-6380 Bad Homburg v.d.H. (DE)
(74) Vertreter: TER MEER STEINMEISTER & PARTNER GbR

(56) Entgegenhaltungen:
- EP-A- 0 226 337
- PHARMAZIE, Band 42, Nr. 5, 1987, Seiten 327-329; M. CAJKOVAC et al.: "Influence of vehicle on antimicrobial efficiency of topical dosage forms of chloramphenicol"
- CHEMICAL ABSTRACTS, Band 104, 1986, Seite 425, Zusammenfassung Nr. 193018j, Columbus, Ohio, US; S. BARTKOWICZ et al.: "Changes in the pharmaceutical and biological availability of aminophylline from suppositories in the presence of nonionic surfactants in the course of one-year storage"

## Beschreibung

Unter ambiphilen Cremes versteht man streichfähige Emulsionen, die als wesentliche Bestandteile eine Fettphase, eine Wasserphase und sowohl einen Wasser-in-Öl- als auch einen Öl-in-Wasser-Emulgator enthalten. Sie zeichnen sich durch extrem feine Phasenverteilung aus. So ist die Ambiphilie am einfachsten dadurch zu erkennen, daß bei mikroskopischen Untersuchungen eine tröpfchenförmige Verteilung der Fettphase in der Wasserphase nicht mehr deutlich erkennbar ist. Unter dem Polarisationsmikroskop erwecken sie den Eindruck eines bikohärenten Phasensystems, d. h., daß Fettphase und Wasserphase nebeneinander vorliegen, keine der beiden Phasen von der anderen eingeschlossen wird. Obwohl man ambiphile Cremes formal zu den hydrophilen Cremesystemen rechnet, sind sie mit lipidartigen Stoffen, wie z. B. Kohlenwasserstoffen, vegetabilischen oder synthetischen Ölen und dergleichen, verdünnbar bzw. mischbar, ebenso wie andererseits stets Mischungsfähigkeit mit Wasser gegeben ist.

Die Mengenanteile der die Fettphase bildenden Lipide bzw. lipidartigen Stoffe liegen im allgemeinen in einem derartigen Bereich, daß die ambiphilen Cremes als eine Art überfettete O/W-Creme betrachtet werden kann.

Ein wesentliches Merkmal der ambiphilen, auch hier neu entwickelten Cremes ist der Gehalt an einem Komplexemulgatorsystem. Hierunter versteht man Gemische aus einem O/W- und einem W/O-Emulgator, wobei vielfach ein schwacher W/O-Emulgator entsprechend höher dosiert wird. Als hydrophiler O/W-Emulgator werden polyoxyalkylierte Ester verwendet, vorzugsweise Polyoxyethylenglycerolmonostearat mit ca. 18 bis ca. 20 Oxyethylengruppen und HLB-Werten im Bereich von 15. Als W/O-Emulgatoren werden insbesondere höhere Fettalkohole, Fettalkoholgemische, Partialester des Glycerols und dergleichen, verwendet. Als Fettalkoholgemische haben sich Cetylstearylalkohole, in denen Cetylalkohol und Stearylalkohol im Mischungsverhältnis von ungefähr 1:1 vorliegen, bewährt. Es können auch Gemische von O/W-Emulgatoren und/oder Gemische von W/O-Emulgatoren eingesetzt werden. So ist die Verwendung von Cetylstearylalkoholen zusammen mit Glycerolmonostearat bekannt, wobei die Stabilität durch das Glycerolmonostearat merklich gefördert wird. Hierbei können auch handelsübliche Glycerolmonostearat-Produkte verwendet werden, die noch Di- und Triglyceride sowie geringe Mengen freies Glycerol und freie Fettsäuren enthalten. Derartige handelsübliche Produkte sind bevorzugt. Als O/W- und W/O-Emulgatoren werden in aller Regel nichtionische Verbindungen eingesetzt. Die Gewichtsverhältnisse von O/W-Emulgator zu W/O-Emulgator liegen im allgemeinen zwischen 1:1 bis 1:2, insbesondere im Bereich von 1:1,5.

Als weiteren Bestandteil enthalten die ambiphilen Cremes bislang Propylenglykol (1,2-Propandiol) als Weichmacher und als Feuchthaltemittel. Propylenglykol hat zudem noch antimikrobielle Eigenschaften.

Ambiphile Cremes sind Gegenstand intensiver Untersuchungen (E. Nürnberg, Arzneimittelforschung 1966, 14, Arzneimittelforschung 1968, 11, und Deutsche Apothekerzeitung, 1968, 907; R. Muckenschnabel, Hydrophile und ambiphile Cremesysteme - Beitrag zur Kenntnis der Struktur und Galenik, Dissertation Erlangen, 1986):

| Muckenschnabel | |
|---|---|
| Glycerolmonostearat | 4,0 g |
| Polyoxyethylenglycerolmonostearat | 7,0 g |
| Mittelkettige Triglyceride | 7,5 g |
| Cetylstearylalkohol | 6,0 g |
| Propylenglykol | 10,0 g |
| Weißes Vaselin | 25,5 g |
| Wasser | 40,0 g |

Zur Vermeidung eines unerwünschten Kristallwachstums der in den Cremes enthaltenen pharmazeutischen Wirkstoffe können Kolloide, insbesondere Hydrokolloide, wie Gelatine bzw. Kollagen oder Methyl- und Hydroxypropylmethylcellulose, enthalten sein.

Auch aus dem DAC ist unter der Bezeichnung Cremor basalis eine ambiphile Creme bekannt:

| Cremor basalis | |
|---|---|
| Glycerolmonostearat | 4,0 g |
| Cetylalkohol | 6,0 g |
| Mittelkettige Triglyceride | 7,5 g |
| Weißes Vaselin | 25,5 g |
| Polyoxyethylenglycerolmonostearat | 7,0 g |
| Propylenglykol | 10,0 g |
| Wasser | 40,0 g |

Der besondere Vorteil der ambiphilen Cremegrundlagen besteht in ihrer Fähigkeit der ungehinderten Liberation von wasserlöslichen und fettlöslichen pharmazeutischen Wirkstoffen.

Es hat sich indessen gezeigt, daß die bekannten ambiphilen Cremes hinsichtlich ihrer Konsistenz und ihren Spreit- und Penetrationseigenschaften noch verbesserungsbedürftig sind, abgesehen von seit einiger Zeit beobachteten Verträglichkeitsproblemen.

Anstelle der häufig Kontaktallergien auslösenden Konservierungsmittel, wie p-Hydroxybenzoesäure, enthalten diese ambiphilen Cremes Propylenglykol als antimikrobielle Substanz, die gleichzeitig als Weichmacher und Feuchthaltemittel wirkt. Diesen galenisch-technischen Vorteilen stehen aber therapeutisch-dermatologische Nachteile gegenüber. Denn auch Propylenglykol hat sensibilisierende und irritierende Wirkungen. Der Versuch, diesen mehrwertigen Alkohol durch Glycerol oder Sorbitol zu ersetzen, hat jedoch, ohne daß dies vorhersehbar gewesen wäre, zum Verlust der Ambiphilie geführt.

Überraschenderweise wurde nunmehr gefunden, daß sich Propylenglykol-unabhängige bzw. -freie ambiphile Cremes dennoch mit einem Gehalt an Glycerol bzw. Sorbitol unter bestimmten, für den Fachmann nicht voraussehbaren Bedingungen herstellen lassen. Eine Voraussetzung für die Verwendung von Glycerol bzw. Sorbitol ist der Verzicht auf Glycerolmonostearat als Komponente des W/O-Emulgatorengemisches, d. h., z. B. die Verwendung nur eines W/O-Emulgators wie z. B. eines aliphatischen Alkohols (Fettalkohol). Diese Tatsache war überraschend, weil gerade das Gemisch von beispielsweise Glycerolmonostearat und Fettalkoholen als besonders günstig für die Rezeptur ambiphiler Cremes angesehen wurde (vgl. vorerwähnte Rezepturen und Literatur). Eine weitere Möglichkeit für die Darstellung ambiphiler Propylenglykol ersetzender Cremes besteht in dem zusätzlichen Gehalt von Isopropylmyristat. Gegenüber der Verwendung von Glycerol bzw. Sorbitol allein handelt es sich hierbei aber auch gleichzeitig um einen alternativen Lösungsweg. Isopropylmyristat kann insbesondere als Komponente der Lipidphase enthalten sein. Bei Anwesenheit von Isopropylmyristat fallen, mit oder ohne Glycerol bzw. Sorbitol, die Beschränkungen im W/O-Emulgator fort, d. h. es kann auch wieder ein W/O-Emulgatorengemisch, z. B. der vorgenannten Art, problemlos eingesetzt werden. Es hat sich überraschenderweise gezeigt, daß ein Gehalt an Isopropylmyristat ganz allgemeiner Anwendung bei ambiphilen Cremes zugänglich ist. Ein solcher Gehalt ermöglicht es, unabhängig von Art und Menge der Emulgatoren, von etwaigen Propylenglkyolgehalten und von sonstigen Bestandteilen nicht nur die Ambiphilie zu verbessern, sondern es ergibt sich auch eine ausgezeichnete Spreitbarkeit und verbesserte Verträglichkeit.

Die erfindungsgemäßen ambiphilen Cremes können Glycerol und/oder Sorbitol, vorzugsweise in Mengen von 1 bis 20 Gew.-%, insbesondere 5 bis 15 Gew.-%, vorzugsweise 10 Gew.-%, enthalten. Dabei hat sich die Gegenwart von Paraffin-Kohlenwasserstoffen besonders bewährt.

Isopropylmyristat als flüssiges Lipid kann z. B. als alleinige Fettphase enthalten sein, was zu besonders weichen Cremes führt. Der Gehalt an Isopropylmyristat beträgt dabei zwischen 20 und 50 Gew.-%, vorzugsweise zwischen 25 und 35 Gew.-%, insbesondere 30 Gew.-%. Neben Isopropylmyristat können, insbesondere innerhalb der vorgenannten, für die gesamte Fettphase geltenden Mengenangaben, auch noch andere bekannte Bestandteile der Fettphase von ambiphilen Cremes verwendet werden, wie flüssiges, insbesondere dickflüssiges Paraffin, Vaseline, mittelkettige Triglyceride, hauptsächlich der Caprin- und Caprylsäure, 2-Octyldodecanol usw., wenn auch dabei darauf zu achten ist, daß dies nicht zu Lasten der Ambiphilie geht. Die Feststellung des Verlusts der Ambiphilie durch Ersatz des Propylenglykols durch Glycerol oder Sorbitol, wenn nicht in der erfindungsgemäßen Weise verfahren wird, und die Erhaltung der Ambiphilie, unabhängig von Propylenglykol, durch Isopropylmyristat ist um so überraschender, als nach der bisherigen Auffassung der Fachwelt die Ambiphilie ausschließlich das Ergebnis der richtigen Zusammenstellung von O/W- und W/O-Emulgatoren ist, nicht dagegen auch von der Natur anderer, z. B. der die Fettphase bildenden Bestandteile abhängig ist. Auf dem erfindungsgemäßen Wege gelingt es, Propylenglykol vollständig oder im gewünschten Umfang zu ersetzen bzw. ambiphile Cremes herzustellen, die gegebenenfalls völlig frei sind von Propylenglykol.

Gegenstand der Erfindung sind ferner ambiphile Cremes mit einem Gehalt an Wasser und Lipiden bzw. lipidartigen Stoffen, nichtionischen O/W- und W/O-Emulgatoren und gegebenenfalls weiteren Bestandteilen, wie Konservierungsmitteln, Spreitmitteln, Weichmachern, Feuchthaltemitteln, Kristallisationsinhibitoren, und/oder pharmazeutischen Wirkstoffen und dergleichen, wobei diese Cremes einen Wassergehalt von 25 bis 55 Gew.-%, insbesondere 38 bis 45 Gew.-% und vorzugsweise 40 bis 43 Gew.-%, einen Gehalt von 1 bis 20 Gew.-%, vorzugsweise 7 bis 15, insbesondere 10 Gew.-% Glycerol und/oder Sorbitol und/oder einen Gehalt an Isopropylmyristat von 20 bis 50 Gew.-%, insbesondere 30 Gew.-%, gegebenenfalls zusammen mit weiteren lipidartigen Stoffen, jeweils bezogen auf die Gesamtmasse der ambiphilen Creme, haben.

Häufig hat sich ein Gehalt an kristallisationsinhibierenden Stoffen zur Verhinderung des Kristallwachstums der Wirkstoffe als vorteilhaft erwiesen. Als derartige Stoffe kommen insbesondere Kolloide, vorzugsweise Gelatine, oder auch andere Hydrokolloide, ohne daß damit andere bekannte Kristallisationsinhibitoren ausgeschlossen werden, in Betracht. Derartige Stoffe, wie Gelatine, können in Mengen von 0,1 bis 5, vorzugsweise 0,3 bis 1 Gew.-% enthalten sein.

Die nachfolgenden Beispiele dienen der Erläuterung der Erfindung.

### Beispiele

| | | | | | | |
|---|---|---|---|---|---|---|
| Polyoxyethylenglycerolmonostearat (Tagat® S2) | 7,0 | 7,0 | 7,5 | 7,0 | 7,5 | 7,5 |
| Glycerolmonostearat (Tegin® M) | 4,0 | 4,0 | 4,0 | 4,0 | - | - |
| Cetylstearylalkohol (Lanette® O) | 6,0 | 6,0 | 6,0 | 6,0 | 10,0 | 10,0 |
| Dickflüssiges Paraffin (PS) | 10,0 | 15,0 | 15,0 | - | 30,0 | 25,0 |
| Isopropylmyristat (IPM) | 20,0 | 5,0 | 7,5 | 20,0 | - | - |
| 2-Octyldodecanol (Eutanol® G) | - | - | 7,5 | 5,0 | - | - |
| Mittelkettige Triglyceride (Miglycol® 812) | - | 10,0 | - | 5,0 | - | - |
| Glycerol | 10,0 | 10,0 | 10,0 | 10,0 | 10,0 | 10,0 |
| Wasser | 42,5 | 43,0 | 42,0 | 42,5 | 41,9 | 47,1 |
| Gelatine | 0,5 | - | 0,5 | 0,5 | 0,5 | - |
| Konservierungsmittel | q.s. | q.s. | q.s. | q.s. | q.s. | q.s. |

## Patentansprüche

1. Ambiphile Creme mit einem Gehalt an Wasser und Lipiden bzw. lipidartigen Stoffen, O/W- und W/O-Emulgatoren und gegebenenfalls weiteren Cremebestandteilen, wie Konservierungsmitteln, Spreitmitteln, Weichmachern, Feuchthaltemitteln, Kristallisationsinhibitoren, indifferenten Hydrokolloiden und/oder pharmazeutischen Wirkstoffen und dergleichen, **dadurch gekennzeichnet,** daß
a) ein Glycerolmonostearat-freier W/O-Emulgator
und Glycerol und/oder Sorbitol enthalten ist
und/oder
b) W/O-Emulgator-unabhängig Isopropylmyristat enthalten ist.

2. Ambiphile Creme gemäß Patentanspruch 1, **dadurch gekennzeichnet,** daß das Glycerol in einer Menge von 1 bis 20 Gew.-%, insbesondere von 5 bis 15 Gew.-% und vorzugsweise von ca. 10 Gew.-%, bezogen auf das Gesamtgewicht der amibphilen Creme, enthalten ist.

3. Ambiphile Creme gemäß Patentanspruch 1 oder 2, **dadurch gekennzeichnet,** daß Isopropylmyristat als Fettphase oder Fettphasenbestandteil enthalten ist.

4. Ambiphile Creme gemäß einem der vorhergehenden Patentansprüche, **gekennzeichnet durch** einen Wassergehalt von 25 bis 55, insbesondere 40 bis 43 Gew.-%, einen Gehalt an Glycerol und/oder Sorbitol von 1 bis 20 Gew.-% und/oder einen Gehalt an Isopropylmyristat, gegebenenfalls zusammen mit anderen Lipiden bzw. lipidartigen Stoffen, von 20 bis 50 Gew.-%, insbesondere 30 Gew.-%, jeweils bezogen auf das Gesamtgewicht der ambiphilen Creme.

5. Ambiphile Creme gemäß Patentanspruch 3, **dadurch gekennzeichnet,** daß als Lipide bzw. lipidartige Stoffe gegebenenfalls neben Isopropylmyristat auch flüssiges Paraffin, Vaseline, Octyldodecanol und/oder mittelkettige Triglyceride enthalten sind.

6. Ambiphile Creme gemäß einem der vorhergehenden Patentansprüche, **dadurch gekennzeichnet,** daß als W/O-Emulgator ein höherer aliphatischer Alkohol oder ein Gemisch mehrerer höherer aliphatischer Alkohole oder ein Gemisch dieser Substanzen mit Partialestern des Glycerols mit höheren aliphatischen Säuren, insbesondere mit Glycerolmonostearat, vorzugsweise in Anteilen von 5 bis 16 Gew.-%, insbesondere von 8 bis 12 Gew.-% und besonders bevorzugt von ca. 10 Gew.-%, bezogen auf das Gesamtgewicht der Creme, enthalten sind.

7. Verwendung von Isopropylmyristat als Bestandteil von ambiphilen Cremes, insbesondere als Fettphase oder Fettphasenbestandteil.

## Claims

1. An ambiphilic cream with a content of water and lipids or lipid-like substances, oil-in-water and water-in-oil emulsifiers and as desired other cream constituents, such as stabilizers, spreading agents, emollients, humectants, crystallization inhibitors, indifferent hydrocolloids and/or active pharmaceuticals and the like, characterized in that
a) it contains a glycerol-monostearate-free water-in-oil emulsifier and glycerol and/or sorbitol and/or
b) it contains isopropyl myristate that is independent of water-in-oil emulsifier.

2. An ambiphilic cream according to claim 1, characterized in that it contains the glycerol in an amount of 1 to 20 weight percent, especially 5 to 15 weight percent and preferably about 10 weight percent relative to the total weight of the ambiphilic cream.

3. An ambiphilic cream according to claim 1 or 2, characterized in that it contains isopropyl myristate as a fatty phase or fatty-phase constituent.

4. An ambiphilic cream according to one of the preceding claims, characterized by a water content of 25 to 55, preferably 40 to 43 weight percent, a content of glycerol and/or sorbitol of 1 to 20 weight percent and/or a content of isopropyl myristate, as desired together with other lipids or lipid-like substances, of 20 to 50 weight percent, especially 30 weight percent, in each case relative to the total weight of the ambiphilic cream.

5. An ambiphilic cream according to claim 3, characterized in that it also contains liquid paraffin, vaseline, octyldodecanol and/or medium-chain-length triglycerides as the lipids and/or lipid-like substances as desired in addition to isopropyl myristate.

6. An ambiphilic cream according to one of the preceding claims, characterized in that it contains, as the water-in-oil emulsifier, a higher aliphatic alcohol or a mixture of a plurality of higher aliphatic alcohols or a mixture of these substances with partial esters of the glycerol with higher aliphatic acids, especially glycerol monostearate, preferably in proportions of 5 to 16 weight percent, especially 8 to 12 weight percent and especially preferably about 10 weight percent, relative to the total weight of the cream.

7. The use of isopropyl myristate as a constituent of ambiphilic creams, especially as a fatty phase or fatty-phase constituent.

## Revendications

1. Crème ambiphile contenant de l'eau et des lipides ou des substances lipidiques, des émulsifiants d'huile dans l'eau et des émulsifiants d'eau dans l'huile et, éventuellement, d'autres ingrédients de crème, comme des agents conservateurs, des agents dispersants, des plastifiants, des agents humidifiants, des inhibiteurs de cristallisation, des hydrocolloïdes neutres et/ou des principes actifs pharmaceutiques et des substances semblables, caractérisée en ce que:
a) elle contient un émulsifiant d'eau dans l'huile, dépourvu de monostéarate de glycérol, et du glycérol et/ou du sorbitol,
et/ou
b) elle contient du myristate d'isopropyle, indépendamment de l'émulsifiant d'eau dans l'huile.

2. Crème ambiphile selon la revendication 1, caractérisée en ce qu'elle contient le glycérol en une quantité valant de 1 à 20% en poids, en particulier de 5 à 15% en poids et, de préférence, d'environ 10% en poids, par rapport au poids total de la crème ambiphile.

3. Crème ambiphile selon la revendication 1 ou 2, caractérisée en ce qu'elle contient le myristate d'isopropyle en tant que phase grasse ou constituant de la phase grasse.

4. Crème ambiphile selon l'une des revendications précédentes, caractérisée par une teneur en eau valant de 25 à 55% en poids, en particulier, valant de 40 à 43% en poids, une teneur en glycérol et/ou en sorbitol valant de 1 à 20% en poids et/ou une teneur en myristate d'isopropyle, éventuellement pris en compte avec d'autres lipides ou substances lipidiques, valant de 20 à 50% en poids, en particulier, valant 30% en poids, chaque teneur étant déterminée par rapport au poids total de la crème ambiphile.

5. Crème ambiphile selon la revendication 3, caractérisée en ce qu'elle contient également, en tant que lipides ou substances lipidiques, éventuellement présent(e)s avec le myristate d'isopropyle, de la paraffine liquide, de la vaseline, de l'octyldodécanol et/ou des triglycérides à chaîne moyenne.

6. Crème ambiphile selon l'une des revendications précédentes, caractérisée en ce qu'elle contient, en tant qu'émulsifiant d'eau dans l'huile, un alcool aliphatique supérieur ou un mélange de plusieurs alcools aliphatiques supérieurs ou un mélange de ces substances avec des esters partiels du glycérol et d'acides aliphatiques supérieurs, en particulier, avec du monostéarate de glycérol, de préférence, dans des proportions valant de 5 à 16% en poids, en particulier, valant de 8 à 12% en poids, une proportion d'environ 10% en poids étant particulièrement préférée, par rapport au poids total de la crème ambiphile.

7. Utilisation de myristate d'isopropyle comme constituant de crème ambiphile, en particulier en tant que phase grasse ou constituant de phase grasse.
